# EUROPEAN PATENT APPLICATION

(11) **EP 4 056 148 A1**
(43) Date of publication of application: **14.09.2022**
(21) Application number: 22159771.9
(22) Date of filing: 02.03.2022
(51) Int. Cl.: A61D 7/00, A61D 9/00, B65D 83/62, A61L 9/14

(54) **PET WOUND SPRAYING APPARATUS**

(30) Priority: 09.03.2021 US 202163158693 P
(71) Applicant: Qianxun Everything (Shenzhen) Technology Co., Ltd., Baoan District Shenzhen Guangdong 518101 (CN)
(72) Inventor: Wang, Rui, Shenzhen, 518101 (CN); Luo, Weilun, Shenzhen, 518101 (CN); Bi, Guoqiao, Shenzhen, 518101 (CN)
(74) Representative: Petraz, Gilberto Luigi

(57) **Abstract**

A spraying apparatus for treating pet wounds and caring for pet skin comprises a spray can, a container containing a hypochlorous acid solution, and a spray nozzle. The container is disposed in the spray can, and a compressed gas is filled between the container and an inner wall of the spray can. The spray nozzle is attached to the spray can and coupled with the container. When activated, the spray nozzle generates a mist from the hypochlorous acid solution.

## Description

### TECHNICAL FIELD

The present application is directed to spraying apparatuses for treating fluids, and more particularly to spraying apparatuses for treating pet wounds and caring for pet skin.

### BACKGROUND

Hypochlorous acid can form non-toxic, colorless, and substantially odorless solutions suitable for sterilization of skin and mucous membranes. Hypochlorous acid solution can also improve the microbial environment around wounds and promote the healing of damaged skin. Current pet care products utilizing hypochlorous acid include spraying apparatuses with plastic trigger spray bottles. However, some pets are sensitive to sudden noises and disturbances, such as the intermittent noise and mist generated by plastic trigger spray bottles. Treatments by such products could provoke or frighten the pets.

Bag-on-valve spraying apparatuses comprise a canister and a bag placed in the canister. The bag is filled with the product to be sprayed. A spray valve is fitted to the canister and is coupled with the bag for spraying out the product. The canister is pressurized with a propellant gas. When the spray valve is activated, the propellant gas pushes the product though the spray valve to form a mist.

### SUMMARY

The present disclosure provides a spraying apparatus for treating pet wounds and caring for pet skin. The spraying apparatus comprises a spray can, a container containing a hypochlorous acid solution, and a spray nozzle. The container is disposed in the spray can, and a compressed gas is filled between the container and an inner wall of the spray can. The spray nozzle is attached to the spray can and coupled with the container. When activated, the spray nozzle generates a mist from the hypochlorous acid solution.

### BRIEF DESCRIPTION OF THE DRAWINGS

FIG. 1 is a perspective view of a spraying apparatus according to an exemplary embodiment.
FIG. 2 is a sectional view of the spraying apparatus shown in FIG. 1.
FIG. 3 is a sectional view of the spraying apparatus shown in FIGS. 1 and 2 in operation.

### DETAILED DESCRIPTION

The invention now will be described more fully hereinafter through reference to various embodiments. These embodiments are provided so that this disclosure convey the scope of the invention to those skilled in the art. Indeed, the invention may be embodied in many different forms and should not be construed as limited to the embodiments set forth herein; rather, these embodiments are provided so that this disclosure will satisfy applicable legal requirements. As used in the specification, and in the appended claims, the singular forms "a", "an", and "the", include plural referents unless the context clearly dictates otherwise.

While various inventive aspects, concepts and features of the inventions may be described and illustrated herein as embodied in combination in the exemplary embodiments, these various aspects, concepts and features may be used in many alternative embodiments, either individually or in various combinations and subcombinations thereof. Unless expressly excluded herein all such combinations and subcombinations are intended to be within the scope of the present inventions. Still further, while various alternative embodiments as to the various aspects, concepts and features of the inventions-such as alternative materials, structures, configurations, methods, devices and components, alternatives as to form, fit and function, and so on-may be described herein, such descriptions are not intended to be a complete or exhaustive list of available alternative embodiments, whether presently known or later developed. Those skilled in the art may readily adopt one or more of the inventive aspects, concepts or features into additional embodiments and uses within the scope of the present inventions even if such embodiments are not expressly disclosed herein. Additionally, even though some features, concepts or aspects of the inventions may be described herein as being a preferred arrangement or method, such description is not intended to suggest that such feature is required or necessary unless expressly so stated. Still further, exemplary or representative values and ranges may be included to assist in understanding the present disclosure, however, such values and ranges are not to be construed in a limiting sense and are intended to be critical values or ranges only if so expressly stated. Parameters identified as "approximate" or "about" a specified value are intended to include both the specified value and values within 10% of the specified value, unless expressly stated otherwise. Further, it is to be understood that the drawings accompanying the present disclosure may, but need not, be to scale, and therefore may be understood as teaching various ratios and proportions evident in the drawings. Moreover, while various aspects, features and concepts may be expressly identified herein as being inventive or forming part of an invention, such identification is not intended to be exclusive, but rather there may be inventive aspects, concepts and features that are fully described herein without being expressly identified as such or as part of a specific invention, the inventions instead being set forth in the appended claims. Descriptions of exemplary methods or processes are not limited to inclusion of all steps as being required in all cases, nor is the order that the steps are presented to be construed as required or necessary unless expressly so stated.

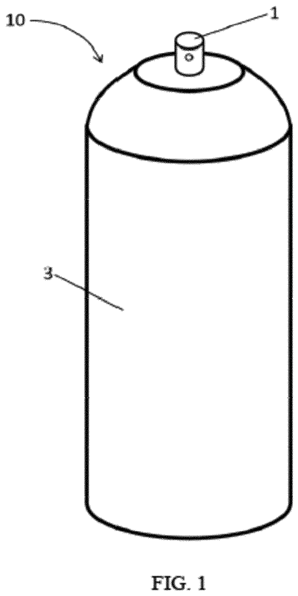

As illustrated in FIGS. 1-3, in some embodiments, a spraying apparatus 10 for treating pet wounds and caring for pet skin comprises a spray can 3, a container 2 containing a hypochlorous acid solution 4, and a spray nozzle 1. The container 2 is disposed in the spray can 3, and a compressed gas 5 is filled between the container 2 and an inner wall of the spray can 3. The spray nozzle 1 is attached to the spray can 3 and is coupled with the container 2. When activated, the spray nozzle 1 sprays out the hypochlorous acid solution 4 to form a mist.

Some pets are sensitive to sudden noises and disturbances. For example, some cats dislike or fear the intermittent noise and mist generated by plastic trigger spray bottles. Treatments by applying a mist generated by such bottles could be difficult when the pets are provoked or frightened. In some embodiments, the spraying apparatus 10 has a bag-on-valve configuration and generates a continuous mist with almost no noise. Therefore, the spraying apparatus 10 is more pet friendly than spraying apparatuses comprising plastic trigger spray bottles.

Bag-on-valve spraying apparatuses require high-strength materials for the spray cans in order to contain compressed gas. In some embodiments of the spraying apparatus 10, the spray can 3 is made from aluminum. Hypochlorous acid is slightly corrosive to aluminum. Also, due to the reaction with aluminum, the cleaning and disinfecting effectiveness of a hypochlorous acid solution decreases over time in an aluminum container. Accordingly, in medical applications, hypochlorous acid solutions are usually stored in plastic containers. In some embodiments, the hypochlorous acid solution 4 is stored in the container 2 disposed in the spray can 3. As such, the hypochlorous acid solution 4 is not directly in contact with the spray can 3. Therefore, although the spray can 3 is made from aluminum, it does not damage the effectiveness of the hypochlorous acid solution 4. Due to the bag-on-valve configuration, the spray 10 generates a homogeneous fine mist of hypochlorous acid solution which enhances the absorption by the pet's skin.

As illustrated in FIGS. 1-3, the spraying apparatus 10 comprises a spray can 3. In some embodiments, the spray can 3 is made from materials having adequate strength for containing the compressed gas in a bag-on-valve spraying apparatus. For example, the spray can 3 may be made from aluminum.

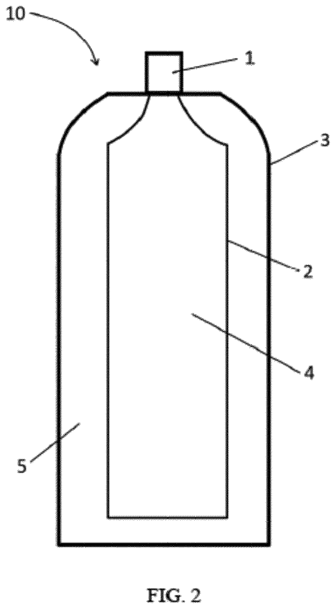

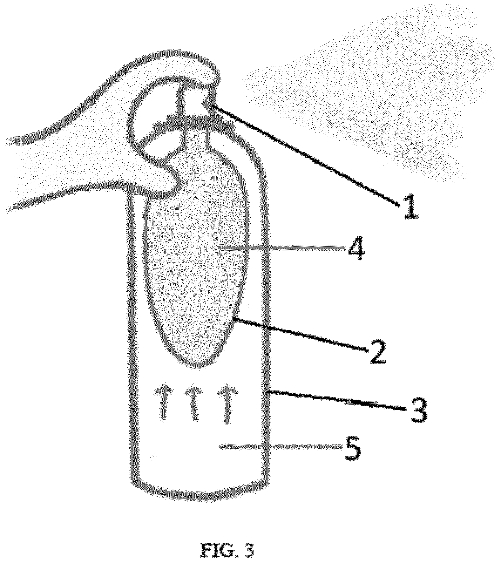

As illustrated in FIGS. 2 and 3, the container 2 contains the hypochlorous acid solution 4, and is disposed inside the spray can 3. The container 2 is made from flexible materials, such that the pressure from the compressed gas 5 is exerted on the hypochlorous acid solution 4. The material of the container 2 is also suitable for storing hypochlorous acid solution. For example, the container 2 may be made from flexible plastic materials such as LDPE, HDPE, polypropylene, PET, nylon, and combinations thereof. In some embodiments, the container 2 is a food grade plastic bag. In some embodiments, the container 2 is a plastic bag made from metalized plastic sheet.

In some embodiments, the hypochlorous acid solution 4 is a water solution of hypochlorous acid. In some embodiments, the hypochlorous acid solution 4 consists of only water and hypochlorous acid. The hypochlorous acid concentration in the hypochlorous acid solution 4 may be any concentration suitable for treating pet wounds, caring for pet skin, and contacting human skin, such as from 0.01% to 0.05% by weight. In some embodiments, the concentration of the hypochlorous acid solution is 0.02% by weight. The hypochlorous acid solution may be injected into the container 2 in an aseptic environment, in order to ensure the cleaning and disinfecting effectiveness.

As illustrated in FIGS. 2 and 3, the compressed gas 5 is filled between the container 2 and the inner wall of the spray can 3. The compressed gas 5 may comprise any gas suitable for a bag-on-valve spraying apparatus, such as nitrogen. The compressed gas 5 exerts pressure on the hypochlorous acid solution 4, such that when the spray nozzle 1 is activated, the hypochlorous acid solution 4 is pushed through the spray nozzle 1 to generate a mist of hypochlorous acid solution.

As illustrated in FIGS. 1-3, the spray nozzle 1 is attached to the spray can 3 and is coupled with the container 2. In some embodiments, the spray nozzle 1 can be activated by the user pressing down the spray nozzle 1. When activated, the spray nozzle 1 allows the hypochlorous acid solution 4 to pass through, and generates a mist of hypochlorous acid solution. Preferably, the spray nozzle generates a fine mist of hypochlorous acid solution to enhance the absorption by the pet's skin. For example, in some embodiments, the droplets in the mist have diameters in the order of magnitude of nanometers. In some embodiments, the spray nozzle 1 can be activated to generate a continuous and homogeneous mist, as the hypochlorous acid solution 4 is constantly under the pressure from the compressed gas 5. In some embodiments, the spray nozzle 1 generates almost no noise when spraying out the hypochlorous acid solution 4. In comparison, conventional aerosol spraying apparatuses with liquefied gas propellants generate more noise due to the propellant flowing out of the spray can. As such, the spraying apparatus 10 is more pet friendly than the conventional aerosol spraying apparatuses.

In operation, the user generates a mist of hypochlorous acid solution by activating the spray nozzle 1. The mist is generated as the hypochlorous acid 4 passes through the spray nozzle 1 under the pressure from the compressed gas 5. Next, the user applies the mist to wounds or the skin of the pet.

In some embodiments, the spraying apparatus 10 comprises an atomizer instead of the spray nozzle 1. The atomizer is attached to the spray can 3 and is coupled with the container 2. When activated, the atomizer generates a mist from the hypochlorous acid solution 4.

In some embodiments, a spraying apparatus for treating pet wounds and caring for pet skin comprises an aluminum spray can. The spraying apparatus can contains a hypochlorous acid solution and a compressed propellant gas. Since hypochlorous acid can react with aluminum, the inside surface of the spray can is coated with a plastic coating to prevent the hypochlorous acid solution from contacting with aluminum. A spray nozzle is attached to the spray can. When activated, the spray nozzle allows the mixture of the hypochlorous acid solution and the propellant gas to flow out from the spray can, generating a mist of hypochlorous acid solution.

## Claims

1. A spraying apparatus for treating pet wounds and caring for pet skin, comprising:
a spray can;
a container containing a hypochlorous acid solution, wherein the container is disposed in the spray can, and wherein a compressed gas is filled between the container and an inner wall of the spray can;
a spray nozzle attached to the spray can and coupled with the container, wherein when activated, the spray nozzle generates a mist from the hypochlorous acid solution.

2. The spraying apparatus according to claim 1, wherein the spray can is made from aluminum.

3. The spraying apparatus according to any one of claims 1 and 2, wherein the container is a food grade plastic bag.

4. The spraying apparatus according to any one of claims 1-3, wherein the hypochlorous acid solution is a water solution of hypochlorous acid having a concentration of 0.02% by weight.

5. The spraying apparatus according to any one of claims 1-4, wherein the hypochlorous acid solution is injected into the container in an aseptic environment.

6. The spraying apparatus according to any one of claims 1-5, wherein the compressed gas comprises nitrogen.

7. The spraying apparatus according to any one of claims 1-6, wherein the mist from the hypochlorous acid solution comprises droplets having diameters in the order of magnitude of nanometers.

8. The spraying apparatus according to any one of claims 1-7, wherein the mist from the hypochlorous acid is generated continuously and homogeneously.

9. The spraying apparatus according to any one of claims 1-8, wherein the spray nozzle generates almost no noise when generating the mist from the hypochlorous acid solution.

10. A method for treating pet wounds and caring for pet skin, comprising:
generating a mist of hypochlorous acid solution; and
applying the mist to wounds or the skin of a pet;
wherein the mist is generated by a spraying apparatus comprising:
a spray can;
a container containing a hypochlorous acid solution, wherein the container is disposed in the spray can, and wherein a compressed gas is filled between the container and an inner wall of the spray can; and
a spray nozzle attached to the spray can and coupled with the container, wherein when activated, the spray nozzle generates the mist from the hypochlorous acid solution.
